# EUROPÄISCHE PATENTANMELDUNG

(11) **EP 0 684 243 A1**
(43) Veröffentlichungstag der Anmeldung: **29.11.1995**
(21) Anmeldenummer: 95106060.7
(22) Anmeldetag: 22.04.1995
(51) Int. Cl.: C07D 473/40

(54) **Verfahren zur Herstellung von 2-Amino-6-chlorpurin und 2-Acylamino-6-chlorpurinen**

(30) Priorität: 30.04.1994 DE 4415196
(71) Anmelder: BOEHRINGER INGELHEIM KG, D-55216 Ingelheim (DE); BOEHRINGER INGELHEIM INTERNATIONAL GmbH, D-55216 Ingelheim am Rhein (DE)
(72) Erfinder: Sobotta, Rainer, Dr. Dipl.-Chem., D-55218 Ingelheim/Rhein (DE); Ramert, Reiner, Dr. Dipl.-Chem., D-55413 Weiler bei Bingen (DE)

(57) **Zusammenfassung**

Die vorliegende Erfindung betrifft ein Verfahren zur Herstellung von 2-Amino-6-chlorpurin und 2-Acylamino-6-chlorpurinen, dadurch gekennzeichnet, daß man ein 2,9- Diacylguanin in Gegenwart eines tertiären Amins und eines Chlorids eines Alkali-oder Erdalkalimetalls mit einem anorganischen Säurechlorid bei einer Reaktionstemperatur in einem Intervall von 60 bis 120°C in einem polaren Lösungsmittel umsetzt, das Reaktionsgemisch nach dem Abkühlen auf eine Temperatur in einem Bereich von 10 bis 30°C mit Wasser hydrolysiert, wobei der pH-Wert des Hydrolysegemisches durch Zugabe einer basisch reagierenden Verbindung im Neutralbereich gehalten wird und gegebenenfalls das beim Abkühlen auskristallisierende 2-Acylamino-6-chlorpurin isoliert oder nach Zugabe der alkalisch reagierenden Verbindung in der Siedehitze deacyliert und nach dem Abkühlen in dem Reaktionsgemisch mit der wässerigen Lösung einer Mineralsäure einen pH-Wert von 7 einstellt und den dabei ausfallenden Niederschlag isoliert und trocknet.

## Beschreibung

Die vorliegende Erfindung betrifft ein neues und verbessertes Verfahren zur Herstellung von 2-Amino-6-chlorpurin und 2-Acylamino-6-chlorpurinen.

2-Amino-6-chlorpurin sowie 2-Acylamino-6-chlorpurine stellen essentielle Zwischenstufen bei der Herstellung von nuclosidanaloger antiviralwirksamer Arzneimittel dar, wie z. B. Penciclovir oder Famciclovir.

Aus dem Stand der Technik sind bereits mehrere Verfahren zur Darstellung derartiger 2-Amino-6-halogenpurine bekannt. So wird in der britischen Patentschrift 767.216 ein Verfahren beschrieben, in dem Guanin zunächst mit Phosphorpentasulfid umgesetzt wird, wodurch eine Mercaptogruppe in die 6-Position des Purinsystems eingeführt wird. Die nachfolgende Behandlung mit Chlor führt im zweiten Reaktionsschritt zu dem entsprechenden 6-Chlorderivat.

Diesem Syntheseweg haftet jedoch u.a. der Nachteil an, daß die Zersetzungsprodukte des Phosphorpentasulfids einen unangenehmen Geruch besitzen, womit zwangsläufig die Gefahr einer nachteiligen Umweltbeeinflussung verbunden ist. Weitere Nachteile dieses Verfahrens ergeben sich aus der geringen Ausbeute und aus dem Umstand, daß das bei dem im ersten Reaktionsschritt anfallenden Thioguanin mutagene Eigenschaften nachgewiesen werden konnten.

In einem anderen Verfahren wird 2-Amino-6-mercaptopurin mittels Methyliodid in das entsprechende 6-Thiomethylderivat überführt; die nachfolgende Behandlung mit Chlor führt - wie im zuvor beschriebenen Verfahren - zum gewünschten 6-Chlorderivat [J. Am. Chem. Soc. 79 (1957) 2185; J. Am. Chem. Soc. 82 (1960) 2633], jedoch können die Nachteile des oben beschriebenen Verfahren auch auf dieser Syntheseroute nicht umgangen werden.

Bei einer prinzipiell anderen Herstellungsmethode wird Guanin mit Phosphoroxychlorid in Gegenwart eines quartären Ammoniumsalzes umgesetzt, womit auf direktem Wege die Herstellung des 2-Amino-6-chlorpurins gelingt [Japanische Offenlegungsschrift Nr.: 227583/1986]. Dieser Verfahrensweg weist jedoch den Nachteil auf, daß - bedingt durch das Lösungsverhalten des Guanins - die Ausbeute lediglich in einem Rahmen von 30 bis 42 % liegt.

Ein neueres Verfahren [EP 0 543 095] beschreibt einen Reaktionsweg über ein andersartiges Zwischenprodukt. Dazu wird Guanin in Gegenwart eines Halogenierungsmittels mit N,N-Dialkylformylverbindungen - wie z. B. N,N-Dimethylformamid, N,N-Diethylformamid, N-Methylformanilid, N,N-Dimethylacetamid, N-Formylpiperazin, N-Formylmorpholin - umgesetzt.

Die nachfolgende Hydrolyse eröffnet auf diesem Wege u.a. einen Zugang zum 2-Amino-6-chlorpurin, das auf der Basis von Ansätzen in Laborgrößenordnung somit in einer maximalen Ausbeute von bis zu 70 % zugänglich sein soll.

Ein weiteres Verfahren zur Herstellung von 2-Amino-6-halogenpurinen ist jüngst in der DE-OS 42 31 036 offenbart worden.

Ebenfalls kürzlich offengelegt wurde ein Verfahren, das von der Guanin-Vorstufe 2,4-Diamino-6-hydroxypyrimidin ausgeht und in an sich bekannter Weise über die Zwischenstufen 2,4-Diamino-6-chlorpyrimidin und 2,4-Diamino-5-nitro-6-chlorpyrimidin verläuft. Letzteres muß katalytisch hydriert und mit einem reaktiven Ameisensäurederivat wie z. B. einem Orthoester cyclisiert werden. Dieses Verfahren weist gegenüber der klassischen Guaninsynthese insbesondere den Nachteil auf, daß nitriert statt nitrosiert werden muß [DE OS 41 42 568].

Ein weiteres jüngst offenbartes Herstellungsverfahren geht von 2,9-Diacetylguanin aus [WO 93/15075]. Dabei wird 2,9-Diacetylguanin u.a. in Gegenwart eines Phasentransferkatalysators - beispielsweise Triethylmethylammoniumchlorid - mit einem Chlorierungsmittel - wie z. B. 2 bis 4 Äquivalenten Phosphoroxychlorid - umgesetzt. Mittels wässeriger Natronlauge erfolgt dann im zweiten Reaktionsschritt die hydrolytische Abspaltung der beiden Acylgruppen. Auf diesem Wege kann das 2-Amino-6-chlorpurin in einer Ausbeute von ca. 75 % erhalten werden.

Neben den schon eingangs erwähnten Nachteilen der aus dem Stand der Technik bekannten Verfahren besteht insbesondere bei den Verfahren, die von Guanin ausgehen, ein weiterer Nachteil darin, daß die Abtrennung des nicht umgesetzten Guanins nur unter Inkaufnahme eines erheblichen Aufwands möglich ist.

Es ist daher die Aufgabe der vorliegenden Erfindung, ein Verfahren zur Herstellung von 2-Amino-6-chlorpurin zur Verfügung zu stellen, das die aus dem Stand der Technik bekannten Nachteile nicht aufweist und das eine aufwendige Abtrennung unumgesetzten Guanins weitestgehend überflüssig macht.

Eine weitere Aufgabe der vorliegenden Erfindung besteht darin, ein Verfahren zur Herstellung von 2-Acylamino-6-chlorpurinen zur Verfügung zu stellen.

Erfindungsgemäß werden die oben erwähnten Aufgaben dadurch gelöst, daß man 2,9-Diacylguanine - insbesondere 2,9-Diacetylguanin - in Gegenwart eines tertiären Amins - vorzugsweise Triethylamin - und dem Chlorid eines Alkali- oder Erdalkalimetalls - vorzugsweise Lithiumchlorid - in einem polarem Lösungsmittel - vorzugsweise Acetonitril - mit einem anorganischen Säurechlorid - vorzugsweise Phosphoroxychlorid - bei erhöhter Temperatur im Bereich von 60 - 120°C - vorzugsweise im Bereich von 70 bis 80°C - umsetzt und das Reaktionsgemisch nach dem Abkühlen auf eine Temperatur im Bereich von 10 bis 30°C - vorzugsweise auf Raumtemperatur (15 bis 25°C) - mit Wasser versetzt, wobei die Temperatur während der Hydrolyse 70°C nicht übersteigen sollte und der pH-Wert der Reaktionsmischung durch Zugabe einer basisch reagierenden Verbindung - wie z. B. mit dem Hydroxid eines Alkali- oder Erdalkalimetalls, gegebenenfalls in wässeriger Lösung - bevorzugt mit konzentrierter Kalilauge oder wässeriger Natronlauge - im Neutralbereich (pH = 6-8) gehalten wird.

Nach dem Abkühlen der Reaktionsmischung läßt man - beispielsweise - das so hergestellte 2-Acetamino-6-chlorpurin auskristallisieren.

Zur Herstellung von 2-Amino-6-chlorpurin kann man nach Zugabe der basisch reagierenden Verbindung bzw. deren wässeriger Lösung im Überschuß in der Siedehitze deacylieren. Nach dem Abkühlen und Abtrennen fester Reaktionsprodukte wird in dem Reaktionsgemisch mit der wässerigen Lösung einer Mineralsäure - vorzugsweise Salzsäure - ein pH-Wert von ca. 7 eingestellt. Der dabei ausfallende Niederschlag wird abgesaugt und anschließend getrocknet.

Gegenüber den bekannten Verfahren weist das erfindungsgemäße Verfahren den Vorteil auf, daß es kein quartäres Ammoniumsalz als Chloridquelle benötigt und mit wesentlich weniger Phosphoroxychlorid auskommt. Von den stattdessen eingesetzten Hilfsreagenzien Lithiumchlorid und Triethylamin kann ersteres im Abwasser verbleiben, letzteres nicht. Triethylamin kann aber leicht durch Destillation aus dem Abwasser entfernt bzw. zurückgewonnen werden. Hier liegt der entscheidende Vorteil des neuen Verfahrens, da quartäre Ammoniumionen, die bei fast allen bisher veröffentlichten Synthesen ausgehend von Guanin eingesetzt werden, ebenfalls nicht in biologischen Kläranlagen abgebaut werden und daher mit erheblichem Aufwand aus den Abwässern entfernt werden müssen.

Die eingangs genannten Aufgaben werden durch die in den Beispielen beschriebenen Verfahren gelöst. Verschiedenartige, andere Ausgestaltungen des Verfahrens werden für den Fachmann aus der vorliegenden Beschreibung ersichtlich. Es wird jedoch ausdrücklich darauf hingewiesen, daß die Beispiele und die diesen zugeordnete Beschreibung lediglich zum Zweck der Erläuterung und Beschreibung vorgesehen und nicht als Einschränkung der Erfindung anzusehen sind.

### Beispiele

### Beispiel 1:

59 g (0,25 mol) 2,9-Diacetylguanin, 11 g (0,25 mol) Lithiumchlorid und 35 ml (0,25 mol) Triethylamin werden unter Feuchtigkeitsausschluß in 550 ml Acetonitril vorgelegt und 47,5 ml (0,5 mol) Phosphoroxychlorid innerhalb von ca. 5 Minuten ohne Kühlung zugetropft. Das Reaktionsgemisch wird anschließend ca. 2 h lang auf Rückflußtemperatur erhitzt, abgekühlt und unter Kühlung zu 500 ml Wasser laufen gelassen, wobei die Temperatur nicht über 70°C steigen darf und der pH-Wert durch Zugabe von konzentrierter Kalilauge auf ca. 6,5 eingestellt wird. Danach kühlt man ab, impft gegebenenfalls an und läßt bei Raumtemperatur über Nacht kristallisieren. Der Niederschlag wird abgesaugt, gründlich mit kaltem Wasser gewaschen und bei ca. 50°C getrocknet. Es werden 47,6 g (90 % d. Th.) des 2-Acetamino-6-chlorpurins in Form schwach braun gefärbter Kristalle mit einem Gehalt (HPLC) von über 99 % erhalten.

### Beispiel 2:

11,8 g (0,05 mol) 2,9-Diacetylguanin, 2,2 g (0,05 mol) Lithiumchlorid und 7,0 ml (0,05 mol) Triethylamin werden in 110 ml Acetonitril unter Feuchtigkeitsausschuß vorgelegt und 9,5 g (0,1 mol) Phosphoroxychlorid ohne Kühlung zulaufen gelassen. Das Reaktionsgemisch wird anschließend 2 h lang auf Rückflußtemperatur erhitzt und wieder auf Raumtemperatur abgekühlt. Unter Kühlung werden bei maximal 30°C Innentemperatur 473,5 g einer 6,5 %igen wässerigen Lösung von Natriumhydroxyd zugetropft. Das Gemisch wird anschließend erneut 2 h lang rückfließend gekocht, abgekühlt, ausgefallene Salze werden abfiltriert und das Filtrat wird mit konz. Salzsäure auf pH=7 gestellt. Der ausgefallene Niederschlag wird nach Rühren über Nacht bei Raumtemperatur abgesaugt, mit Wasser gewaschen und bei ca. 50°C im Vakuum getrocknet. Es werden 7,6 g (89,8 % d. Th.) hellbraunes Kristallpulver erhalten, das gemäß HPLC ca. 94 % 2-Amino-6-chlorpurin enthält.

### Beispiel 3:

59 g (0,25 mol) 2,9-Diacetylguanin, 11 g (0,25 mol) Lithiumchlorid und 35 ml (0,25 mol) Triethylamin werden unter Feuchtigkeitsausschuß in 550 ml Acetonitril vorgelegt und 47,5 ml (0,5 mol) Phosphoroxychlorid ohne Kühlung innerhalb von 5 min. zugetropft. Das Reaktionsgemisch wird anschließend 2 h lang rückfließend gekocht, auf Raumtemperatur abgekühlt und unter Eiskühlung zu einer Lösung von 300 ml (3,75 mol) 47 %iger Kalilauge in 200 ml Wasser zulaufen gelassen, wobei eine Innentemperatur von 45°C nicht überschritten werden soll.
Die entstandene Suspension wird noch 2 h bei 45-50°C gerührt, danach mit 110 ml (1,33 mol) 37 %iger Salzsäure auf pH=7 gestellt und auf Raumtemperatur abkühlen gelassen. Der Niederschlag wird abgesaugt, gründlich mit kaltem Wasser gewaschen und bei ca. 50°C getrocknet.
Man erhält 38,0 g (89,8 % d. Th.) hellbraun gefärbtes Kristallpulver von 2-Amino-6-chlorpurin mit einem Gehalt (HPLC) von über 99 %.

### Beispiel 4: (Vergleichsbeispiel):

35,4 g (0,15 mol) 2,9-Diacetylguanin, 42 ml (0,30 mol) Triethylamin und 20,7 g (0,15 mol) Triethylaminhydrochlorid werden in 330 ml Acetonitril unter Feuchtigkeitsausschluß vorgelegt und 28,5 ml (0,3 mol) Phosphoroxychlorid innerhalb von 1 min. zulaufen gelassen. Das Reaktionsgemisch wird 2 h rückfließend gekocht, dann abgekühlt und unter Einsatz von 300 ml Wasser und 100 ml konzentrierter Kalilauge aufgearbeitet wie in Beispiel 1 beschrieben. Es werden 22,0 g (69,3 % d. Th.) eines dunkelbraunen Kristallpulvers erhalten, das gemäß HPLC ca. 99 % 2-Acetamido-6-chlorpurin enthält.

## Patentansprüche

1. Verfahren zur Herstellung von 2-Amino-6-chlorpurin, dadurch gekennzeichnet, daß man ein 2,9-Diacylguanin in Gegenwart eines tertiären Amins und eines Chlorids eines Alkali- oder Erdalkalimetalls mit einem anorganischen Säurechlorid bei einer Reaktionstemperatur in einem Intervall von 60 bis 120°C in einem polaren Lösungsmittel umsetzt, das Reaktionsgemisch nach dem Abkühlen auf eine Temperatur in einem Bereich von 10 bis 30°C mit Wasser hydrolysiert, wobei der pH-Wert des Hydrolysegemisches durch Zugabe einer basisch reagierenden Verbindung im Neutralbereich gehalten wird und gegebenenfalls das beim Abkühlen auskristallisierende 2-Acylamino-6-chlorpurin isoliert oder nach Zugabe der alkalisch reagierenden Verbindung in der Siedehitze deacyliert und nach dem Abkühlen in dem Reaktionsgemisch mit der wässerigen Lösung einer Mineralsäure einen pH-Wert von 7 einstellt und den dabei ausfallenden Niederschlag isoliert und trocknet.

2. Verfahren zur Herstellung von 2-Amino-6-chlorpurin, dadurch gekennzeichnet, daß man 2,9-Diacetylguanin in Gegenwart von Triethylamin und Lithiumchlorid mit Phosphoroxychlorid bei einer Reaktionstemperatur in einem Intervall von 70 bis 80°C in Acetonitril umsetzt, das Reaktionsgemisch nach dem Abkühlen auf eine Temperatur in einem Bereich von 15 bis 25°C mit Wasser hydrolysiert, wobei der pH-Wert des Hydrolysegemisches durch Zugabe von wässeriger Natron- oder Kalilauge in einem Bereich von 6-8 gehalten wird und gegebenenfalls das beim Abkühlen auskristallisierende 2-Acetylamino-6-chlorpurin isoliert oder nach Zugabe der alkalisch reagierenden Verbindung in der Siedehitze deacyliert und nach dem Abkühlen in dem Reaktionsgemisch mit wässeriger Salzsäure-Lösung einen pH-Wert von 7 einstellt und den dabei ausfallenden Niederschlag isoliert und trocknet.
